## Europäisches Patentamt
## European Patent Office
## Office européen des brevets

(11) Publication number: **0 017 292**
**B1**

(12) # EUROPEAN PATENT SPECIFICATION

(45) Date of publication of patent specification: **06.10.82**

(51) Int. Cl.³: **C 07 D 201/08,**
**C 07 D 207/267 //B01J23/78**

(21) Application number: **80200283.2**

(22) Date of filing: **27.03.80**

(54) Process for the preparation of an optionally C-substituted 2-pyrrolidone.

(30) Priority: **01.04.79 NL 7902537**

(43) Date of publication of application:
**15.10.80 Bulletin 80/21**

(45) Publication of the grant of the patent:
**06.10.82 Bulletin 82/40**

(84) Designated Contracting States:
**BE DE FR GB IT NL**

(56) References cited:
**DE - B - 1 014 113**
**FR - A - 2 008 643**
**FR - A - 2 085 095**
**FR - A - 2 316 228**
**FR - A - 2 387 953**
**FR - A - 2 415 100**
**GB - A - 1 240 151**
**US - A - 3 095 423**
**US - A - 4 036 836**
**US - A - 4 123 438**

(73) Proprietor: **STAMICARBON B.V.**
**Postbus 10**
**NL-6160 MC Geleen (NL)**

(72) Inventor: **Beekhuis, Gerrit Ernst**
**Margrietlaan 9**
**NL-6165 CR Geleen (NL)**
Inventor: **van de Mond, Theodorus Johannes**
**Oranjelaan 10**
**NL-6166 BR Geleen (NL)**
Inventor: **Nieuwkamp, Johannes Gerardus Maria**
**Grootveldstraat 11**
**NL-6141 LV Limbricht (NL)**

(74) Representative: **Pinckaers, August René et al,**
**OCTROOIBUREAU DSM Postbus 9**
**NL-6160 MA Geleen (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Process for the preparation of an optionally C-substituted 2-pyrrolidone

The invention relates to a process for the preparation of an optionally C-substituted 2-pyrrolidone by subjecting a substituted or non-substituted succinonitrile to catalytic hydrogenation in the liquid phase, in the presence of ammonia, and treating the resulting hydrogenated product with water.

This known process (see U.S. Patent Specification No. 4,123,438) can be carried out to give a good yield by suspending the catalyst required for the hydrogenation, e.g. Raney nickel, in the liquid phase. In this process the removal of the catalyst after completion of the hydrogenation, e.g. by filtration, is a rather costly operation.

Now a mode of effecting this hydrogenation has been found which can give a good yield together with a less costly separation of catalyst and reaction mixture.

The process according to the invention for the preparation of an optionally C-substituted 2-pyrrolidone by subjecting a substituted or non-substituted succinonitrile to catalytic hydrogenation in the liquid phase, in the presence of ammonia, and treating the resulting hydrogenated product with water is characterized in that the hydrogenation is effected with a catalyst in the form of a fixed bed, this catalyst being nickel on a carrier consisting of one or more compounds from the group formed by calcium hydroxide, calcium carbonate, magnesium oxide, magnesium hydroxide and magnesium carbonate.

The nickel content of the catalyst is, for instance, 5—80% by weight. By preference, a nickel content of 10—60% wt. is applied.

The nickel may be applied to the carrier by known methods, for instance the method according to which the carrier material is impregnated with a solution of a nickel salt, after which the solvent is evaporated, and the nickel salt, if necessary subsequent to having been decomposed to nickel oxide, is reduced to metallic nickel. According to another known method the catalyst can be prepared by precipitating, at a temperature between, e.g. 60 and 100°C, nickel hydroxide and/or nickel carbonate from an aqueous solution of a nickel salt, for instance nickel nitrate, nickel sulphate, nickel chloride, nickel acetate, or nickel formiate, with a concentration of, for instance, between 0.5 and 5 moles of nickel salt per litre, with the aid of a hydroxide and/or carbonate of an alkali metal or ammonium, or with the aid of a urea solution. The resulting precipitate can then be mixed with the carrier material. By preference the carrier material is present already in the nickel salt solution when the nickel hydroxide or nickel carbonate is precipitated. The nickel salt in the resulting mixture is then reduced to metallic nickel, whether or not after decomposition to nickel oxide.

The quantity of ammonia that should be present in the liquid phase per gram of succinonitrile to be converted may vary for instance between 0.5 and 25 grams of ammonia per gram of succinonitrile to be converted. In addition to ammonia, another solvent may be present, for instance toluene, xylene, tetrahydrofuran, pyrrolidone or pyridine, but this does not bring any advantage. Preferably, a solution in liquid ammonia of the succinonitrile to be converted is contacted with the fixed catalyst bed.

The process according to the invention can be carried out at various partial hydrogen pressures, for instance a partial hydrogen pressure between 100 and 35,000 kPa. In practice, a partial hydrogen pressure of 500—10,000 kPa is best suited.

In the process according to the invention the hydrogenation can be carried out at various temperatures, for instance a temperature between 40 and 150°C. By preference a temperature between 50 and 130°C is applied.

The hydrogenation according to the invention can be carried out very suitably in a so-called trickle-phase reactor in which a solution of the succinonitrile in liquid ammonia flows across the fixed catalyst bed — consisting of catalyst material in the form of tablets, granules, extrudates, or pellets — under the influence of gravity, whilst the hydrogen or the hydrogencontaining gas is passed across the catalyst bed co-currently or counter-currently. The specific loading of the catalyst may then have any of various values, e.g. between 0.1 and 25 litres of liquid per litre of catalyst and per hour, by preference between 0.5 and 10 litres of liquid per litre of catalyst and per hour.

After the hydrogenation of the succinonitrile, all or part of the ammonia may be removed from the resulting reaction mixture, e.g. by evaporation. The treatment of the hydrogenated product with water may be carried out in the presence as well as in the absence of ammonia. As in the known process, various temperatures may be applied in this water treatment, for instance a temperature between 150 and 300°C. Also the quantity of water may be varied, as in the known process, for instance between the amount stoichiometrically needed and 20 moles per mole of succinonitrile.

The starting product used in the process according to the invention may be succinonitrile or a substituted succinonitrile, for instance succinonitrile with an alkyl group containing 1—4 carbon atoms substituted in position(s) 2 and/or 3.

The resulting pyrrolidone can, in practice, be used for various conversion processes, for instance the conversion to N-vinyl pyrrolidone from 2-pyrrolidone.

The process according to the invention will be elucidated in the following examples.

### Example I
Catalyst preparation

2000 g of nickel nitrate $(Ni(NO_3)_2 . 6H_2O)$ was dissolved in 4 litres of distilled water. In the resulting solution 270 g of powdery calcium hydroxide (made by J. T. Baker chemicals) was suspended. The suspension was heated to 90°C whilst being stirred, after which in about 1 hour's time a solution of 850 g of anhydrous sodium carbonate in 4 litres of distilled water was added to the suspension. The resulting precipitate was separated off by filtration and washed with a solution of calcium hydroxide in distilled water saturated at room temperature, until the washing water could no longer be demonstrated to contain sodium by the zinc uranyl nitrate method. Thereafter the catalyst mass was dried in air for 24 hours at 120°C.

When analyzed, the mass obtained in this way proved to consist of 32.4% by weight of nickel in the form of nickel carbonate and nickel hydroxide on a mixture of calcium hydroxide and calcium carbonate (weight ratio about 1:1).

After grinding, the mass was mixed with 2% wt. of graphite (as lubricant) and compressed to tablets having a diameter and length of about 3 mm.

Pyrrolidone preparation

Per hour, an amount of 0.1 kg of succinonitrile was dissolved in 1.25 kg of liquid ammonia at elevated pressure in a mixer heated at 80°C, after which the resulting solution was pumped into the top of a vertically disposed metal tubular reactor (length 1.5 metres, internal diameter 2.54 centimetres).

The reactor contained a bottom layer of 600 millilitres of catalyst, and a top layer consisting of 75 millilitres of inert packing material (protruded metal packing, dimensions 0.16 by 0.16 cm). Before the start of the experiment the catalyst had been activated by passing nitrogen across it, for 10 hours and at 200°C, and then hydrogen, for 30 hours and at 335°C.

At the same time as the ammonical solution, hydrogen was introduced into the top of the tubular reactor by means of a compressor, at the rate of 930 litres (0°C and 100 kPa) per hour. The hydrogen partial pressure in the reactor was kept at 4000 kPa. (The total pressure was 10,000 kPa). The temperature in the reactor was maintained at 85°C with the use of a heating jacket.

The resulting reaction mixture was discharged from the reactor at the bottom, cooled to 40°C, and separated under pressure in a separator into liquid and gas. Thereafter, the ammonia was removed from the resulting liquid in an expansion vessel operated at atmospheric pressure. After 25 hours' operation, the next 2 hours product thus obtained was collected. A 2-gram sample of this product (208 grams) was analyzed by gas chromatography, which showed that no starting product was present any more. Whilst being stirred, the remaining amount of the collected product was heated for 0.5 hour at 210°C in a 1-litre autoclave, together with 200 grams of water. After cooling the hydrolyzed product was analyzed by gas chromatography. This showed 185 grams of pyrrolidone to have formed. Calculated in relation to the amount of succinonitrile introduced, this means that the efficiency was 87%. By distillation of the hydrolysis mixture at reduced pressure, virtually pure (99% purity) pyrrolidone can be recovered, which can be further purified, if so desired, by distillation over an acid, for instance sulphuric acid, and/or an alkali, for instance sodium hydroxide.

When the experiment was repeated with a commercially obtainable nickel catalyst (50% wt. nickel on an $Al_2O_3$ carrier) under otherwise equal conditions, the efficiency was 75%.

### Example II
Catalyst preparation

2000 g of nickel nitrate $(Ni(NO_3)_2 . 6H_2O)$ was dissolved in 4 litres of distilled water. In the resulting solution 270 g of powdery magnesium oxide (made by Saline Lüneburg) was suspended. The suspension was heated to 90°C whilst being stirred, after which in about 1 hour's time a solution of 850 g of anhydrous sodium carbonate in 4 litres of distilled water was added to the suspension. The resulting precipitate was separated off by filtration and washed with distilled water until the washing water could no longer be demonstrated to contain sodium by the zinc uranyl nitrate method. Thereafter the catalyst mass was dried in air for 24 hours at 120°C.

When analyzed, the mass obtained in this way proved to consist of 32.7% by weight of nickel in the form of nickel carbonate and nickel hydroxide on magnesium hydroxide.

After grinding, the mass was mixed with 2% by weight of graphite and compressed to tablets having a diameter and length of about 3 mm.

Pyrrolidone preparation

Per hour, an amount of 225 g of succinonitrile was dissolved in 3.1 kg of liquid ammonia at elevated pressure in a mixer heated at 97°C, after which the resulting solution was pumped into the top of a vertically disposed metal tubular reactor (length 1.5 metres, internal diameter 2.54 centimetres).

The reactor contained a layer of 600 millilitres of catalyst, with, on top of it, a layer of 75 millilitres of inert packing material (protruded metal packing, dimensions 0.16 by 0.16 cm). Before the start of the experiment the catalyst had been activated by passing nitrogen across it, for 10 hours and at 200°C, and then hydrogen, for 30 hours and at 335°C.

At the same time as the ammoniacal solution, hydrogen was introduced into the top of the tubular reactor by means of a compressor, at the rate of 1200 litres (0°C and 100 kPa) per hour. The partial hydrogen pressure in the reactor was kept at 3500 kPa. (The total pressure was 10,000 kPa). The temperature in the reactor was maintained at 100°C with the use of a heating jacket.

The resulting reaction mixture was discharged from the reactor at the bottom, cooled to 40°C, and separated under pressure in a separator into liquid and gas. Thereafter the ammonia was removed from the resulting liquid in an expansion vessel operated at atmospheric pressure. After 25 hours' operation, the next 2 hours product thus obtained was collected. A 2-gram sample of this product (245 grams) was analyzed by gas chromatography, which showed that 97% of the starting product had been converted. Whilst being stirred, the remaining amount of the collected product was heated for 45 minutes at 210°C in a 1-litre autoclave, together with 250 gram of water. After cooling, the hydrolized product was analyzed by gas chromatography. This showed 197 grams of pyrrolidone to have formed.

Calculated in relation to the amount of succinonitrile converted, this means that the efficiency was 85%. By distillation of the hydrolysis mixture at reduced pressure, virtually pure (99% purity) pyrrolidone can be recovered, which can be further purified, if so desired, by distillation over an acid, for instance sulphuric acid, and/or alkali, for instance sodium hydroxide.

When the experiment was repeated with a commercially obtainable nickel catalyst (50% wt. nickel on an $Al_2O_3$ carrier) under otherwise equal conditions, the efficiency was 75%.

## Claims

1. Process for the preparation of an optionally C-substituted 2-pyrrolidone by subjecting a substituted or non-substituted succinonitrile to catalytic hydrogenation in the liquid phase, in the presence of ammonia, and treating the resulting hydrogenated product with water, this process being characterized in that the hydrogenation is effected with a catalyst in the form of a fixed bed, and this catalyst being nickel on a carrier consisting of one or more compounds from the group formed by calcium hydroxide, calcium carbonate, magnesium oxide, magnesium hydroxide and magnesium carbonate.

2. Process according to claim 1, characterized in that the nickel content of the catalyst is 10—60% by weight.

3. Process according to any one of the claims 1—2, characterized in that in the hydrogenation 0.5—25 grams of ammonia is used per gram of succinonitrile to be converted.

4. Process according to any one of the claims 1—3, characterized in that the hydrogenation is effected at a partial hydrogen pressure between 500 and 10.000 kPa.

5. Process according to any one of the claims 1—4, characterized in that the hydrogenation is effected at a temperature between 50 and 130°C.

6. Process according to any one of the claims 1—5, characterized in that the hydrogenation is effected in a trickle-phase reactor, and that a specific catalyst loading of between 0.5 and 10 litres of liquid per litre of catalyst and per hour is applied.

## Patentansprüche

1. Verfahren zum Herstellen eines etwaigen C-substituierten 2-Pyrrolidons, indem substituiertes oder nichtsubstituiertes Succinonitril in Anwesenheit von Ammoniak einer katalytischen Hydrierung in der Flüssigphase unterzogen wird, wonach das anfallende hydrierte Produkt mit Wasser behandelt wird, dadurch gekennzeichnet, dass die Hydrierung mit einem Katalysator in Form eines Festbetts vorgenommen wird und der Katalysator Nickel auf einem Träger ist, der aus einer oder mehreren Verbindungen aus der Gruppe Calciumhydroxid, Calciumkarbonat, Magnesiumoxid, Magnesiumhydroxid und Magnesiumkarbonat besteht.

2. Verfahren nach Anspruch 1, dadurch gekennzeichnet, dass der Nickelgehalt des Katalysators 10 bis 60 gew.-% ist.

3. Verfahren nach einem der Ansprüche 1 oder 2, dadurch gekennzeichnet, dass für die Hydrierung 0,5 bis 25 g Ammoniak je g umzuwandelndes Succinonitril verwendet werden.

4. Verfahren nach einem der Ansprüche 1 bis 3, dadurch gekennzeichnet, dass die Hydrierung bei einem Partialwasserstoffdruck zwischen 500 und 10.000 kPa vorgenommen wird.

5. Verfahren nach einem der Ansprüche 1 bis 4, dadurch gekennzeichnet, dass die Hydrierung bei einer Temperatur zwischen 50 und 130°C vorgenommen wird.

6. Verfahren nach einem der Ansprüche 1 bis 5, dadurch gekennzeichnet, dass die Hydrierung in einem Rieselreaktor vorgenommen wird und dass eine spezifische Katalysatorladung von 0,5 bis 10 l Flüssigkeit je l Katalysator und je Stunde verwendet werden.

## Revendications

1. Procédé de préparation d'une 2-pyrrolidone éventuellement substituée en C, en soumettant du succinonitrile substitué ou non à une hydrogénation catalytique en phase liquide, en présence d'ammoniaque, et en traitant le produit hydrogéné obtenu à l'aide d'eau, caractérisé en ce que l'hydrogénation est effectuée à l'aide d'un catalyseur sous forme d'un lit fixe, ce catalyseur étant du nickel sur un support, constitué d'un ou de plusieurs composés du groupe formé par l'hydroxyde de calcium, le carbonate de calcium, l'oxyde de magnésium, l'hydroxyde de magnésium et carbonate de magnésium.

2. Procédé selon la revendication 1, caractérisé en ce que le catalyseur a une teneur en nickel de 10—60% en poids.

3. Procédé selon l'une des revendications 1 et 2, caractérisé en ce qu'on utilise pour l'hydrogénation, 0,5—25 g d'ammoniaque par gramme de succinonitrile à convertir.

4. Procédé selon l'une des revendications 1 à 3, caractérisé en ce que l'hydrogénation est effectuée à une pression partielle d'hydrogène située entre 500 et 10.000 kPa.

5. Procédé selon l'une des revendications 1 à 4, caractérisé en ce que l'hydrogénation est effectuée à une température située entre 50 et 130°C.

6. Procédé selon l'une des revendications 1 à 5, caractérisé en ce que l'hydrogénation est effectuée dans un réacteur à "trickle-phase" et qu'on applique une charge spécifique de catalyseur de 0,5 à 10 litres de liquide par litre de catalyseur et par heure.